# EUROPEAN PATENT APPLICATION

(11) **EP 3 992 276 A1**
(43) Date of publication of application: **04.05.2022**
(21) Application number: 20832378.2
(22) Date of filing: 26.06.2020
(51) Int. Cl.: C12M 3/00, C12N 5/00

(54) **CELL SCAFFOLD MATERIAL, CELL CULTURING SUPPORT, AND CELL CULTURING METHOD**

(30) Priority: 27.06.2019 JP 2019120033
(71) Applicant: Showa Denko Materials Co., Ltd., Tokyo 100-6606 (JP)
(72) Inventor: JOHMEN Masayoshi, Tokyo 1006606 (JP); OJI Masashi, Tokyo 1006606 (JP); ITO Hiroshi, Tokyo 1006606 (JP); FUKUSHIMA Kazuki, Yamagata-shi, Yamagata 990-8560 (JP); SANO Mai, Yamagata-shi, Yamagata 990-8560 (JP)
(74) Representative: Winter, Brandl - Partnerschaft mbB
(86) International application number: PCT/JP2020/025209
(87) International publication number: WO 2020/262609

(57) **Abstract**

A cell scaffold material comprising a copolymer having a polylactic acid structural unit (A) and a polycarbonate structural unit (B). The polycarbonate structural unit (B) may include at least one unit having an alkoxyalkyloxycarbonyl group as a substituent. Also provided is a cell culture support comprising this cell scaffold material and a substrate.

## Description

### TECHNICAL FIELD

Embodiments of the present disclosure relate to a cell scaffold material, a cell culture support, and a cell culture method.

### BACKGROUND ART

Cell culturing is conducted by inoculating a substrate with the cells, and adding a medium. One know method used to enhance the adhesion between the substrate and the cells involves coating the substrate with a cell scaffold material.

In the field of regenerative medicine, which has expanded significantly in recent years, research is being conducted into methods in which stem cells typified by iPS cells and the like are grown by in vitro cell culture, and then used within the body of animals or humans. In cell culturing, cell proliferation is achieved while successive cell subcultures are conducted, and it is preferable that cells are grown for which cell differentiation is suppressed. If a portion of the cells differentiate, cell proliferation progress is hindered, and particularly in the case of large-scale subcultures, cell proliferation may sometimes be inhibited. Further, in those cases where cells having different differentiation stages exist within the culture system, cells from a specific differentiation stage may sometimes require isolation, but isolating these cells with a high degree of purity is technically difficult.

Furthermore, in cell culturing, a protein component can be used as a medium or scaffold material or the like. If this protein becomes incorporated in the final cultured cell product, then the protein can sometimes act as an antigen. Furthermore, when the protein is animal-derived or human-derived, fluctuation between lots can be large, which can sometimes have an effect on stable culturing of the cells. Accordingly, demands are increasing for cell culturing in serum-free media. In particular, there is growing demand for cell scaffold materials that are suitable for stem cell culturing in serum-free media.

Up until now, laminin and fibronectin and the like have been investigated as cell scaffold materials for use in stem cell culturing, but adequate performance has not been obtainable.

JP 2018-068192 A discloses that a compound having a structure formed from an aromatic ring and a hydrogen bonding unit positioned between the blocks of an amphiphilic block copolymer affects cell proliferation and morphological control. Examples of specific compounds disclosed in JP 2018-068192 A include compounds having an N-(4-(ureidomethyl)benzyl)benzamide structure between the blocks of a block copolymer of polyethylene glycol (PEG) and poly-L-lactic acid (PLLA), poly-ε-caprolactone (PCL) or trimethylene carbonate (PTMC).

JP 2018-068192 A proposes the use of these compounds as cell spreading and growth control agents by addition to the culture medium.

### SUMMARY OF INVENTION

### PROBLEMS INVENTION AIMS TO SOLVE

JP 2018-068192 A only discloses applications of these compounds as cell spreading and growth control agents that are added to the culture medium, and little investigation was conducted into cell scaffold materials.

Cell scaffold materials require favorable adhesion between the substrate and cells, while having no adverse effects on cell proliferation. Moreover, in cell culturing using a cell scaffold material, it is sometimes desirable to promote cell proliferation while suppressing differentiation.

The present disclosure has the objects of providing a cell scaffold material, a cell culture support and a cell culture method that suppress cell differentiation and promote cell proliferation.

### MEANS FOR SOLUTION OF THE PROBLEMS

Specific aspects for achieving the above objects are as described below.
[1] A cell scaffold material comprising a copolymer having a polylactic acid structural unit (A) and a polycarbonate structural unit (B).
[2] The cell scaffold material according to [1], wherein the polycarbonate structural unit (B) includes at least one unit having an alkoxyalkyloxycarbonyl group as a substituent.
[3] The cell scaffold material according to [1] or [2], wherein the polycarbonate structural unit (B) includes at least one unit having a methoxyethyloxycarbonyl group as a substituent.
[4] The cell scaffold material according to [1], wherein the polycarbonate structural unit (B) includes at least one unit represented by general formula (I) shown below. 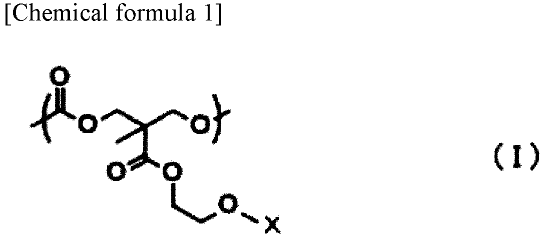 (In general formula (I), X represents a hydrogen atom or an alkyl group having not more than 5 carbon atoms.)
[5] The cell scaffold material according to [4], wherein X in general formula (I) is a methyl group.
[6] The cell scaffold material according to any one of [1] to [5], wherein the copolymer is a block copolymer in which one terminal or both terminals have the polylactic acid structural unit (A).
[7] The cell scaffold material according to [6], wherein the copolymer is an AB-type, ABA-type or ABAB-type block copolymer.
[8] The cell scaffold material according to [7], wherein the copolymer is an ABA-type block copolymer.
[9] The cell scaffold material according to any one of [1] to [8], wherein the polylactic acid structural unit (A) is a poly-D-lactic acid structural unit or a poly-L-lactic acid structural unit.
[10] A cell culture support comprising the cell scaffold material according to any one of [1] to [9], and a substrate.
[11] A cell culture method that comprises: preparing the cell scaffold material according to any one of [1] to [9], placing the cell scaffold material in a culture system, and culturing cells in the presence of the cell scaffold material.
[12] The cell culture method according to [11], wherein the culture system comprises a serum-free medium.

### EFFECTS OF THE INVENTION

The present disclosure provides a cell scaffold material, a cell culture support and a cell culture method that suppress cell differentiation and promote cell proliferation.

### BRIEF DESCRIPTION OF THE DRAWINGS

FIG. 1 is a graph illustrating the fluorescent intensity of fluorescent-labeled BSA when supports coated with various polymers are used.
FIG. 2 is a series of fluorescence difference microscope photographs of normal human fibroblasts cultured using supports coated with various polymers, the photographs showing the states (a) one hour after the start of cell culturing, (b) one day after the start of cell culturing, (c) two days after the start of cell culturing, (d) three days after the start of cell culturing, and (e) seven days after the start of cell culturing.
FIG. 3 is a graph showing the number of cells relative to the cell culture time of normal human fibroblasts cultured using supports coated with various polymers.
FIG. 4 is a graph showing the FGF-2 concentration relative to the cell culture time of normal human fibroblasts cultured using supports coated with various polymers.

### EMBODIMENTS FOR CARRYING OUT THE INVENTION

Embodiments of the present disclosure are described below, but the present disclosure is not limited to the following embodiments.

A cell scaffold material according to one of embodiments comprises a copolymer having a polylactic acid structural unit (A) and a polycarbonate structural unit (B).

This embodiment can suppress cell differentiation and promote cell proliferation.

To explain further, the polylactic acid structural unit and the polycarbonate structural unit that constitute the cell scaffold material according to the present disclosure both have favorable adhesion to cells and high biocompatibility. On the other hand, the polylactic acid structural unit has a hydrophobic property, whereas the polycarbonate structural unit, despite being water-insoluble, has a hydrophilic property compared with the polylactic acid structural unit. Based on these properties, due to the respective characteristics of the structural units, the copolymer has a tendency to self-organize while maintaining adhesiveness to the cells. It is surmised that when the copolymer is used as a cell scaffold material, this structural characteristic has the effects of suppressing cell differentiation and promoting cell proliferation. However, the present disclosure is not constrained by this theory.

The cell scaffold material according to one of embodiments preferably comprises a copolymer having a polylactic acid structural unit (A) and a polycarbonate structural unit (B). This copolymer is preferably a block copolymer of the structural unit (A) and the structural unit (B).

In the copolymer according to one of embodiments, the polylactic acid structural unit (A) means a structural unit formed from a polylactic acid structure, and may be a structural unit containing a plurality of lactic acid units.

Examples of the polylactic acid structural unit (A) include poly-D-lactic acid structural units, poly-L-lactic acid structural units, and poly-DL-lactic acid structural units. The polylactic acid structural unit (A) is preferably a poly-D-lactic acid structural unit or a poly-L-lactic acid structural unit, and is more preferably a poly-D-lactic acid structural unit. In those cases where the copolymer contains two or more polylactic acid structural units (A), the polylactic acid structural units (A) contained in a single molecule of the copolymer may all be the same, or some or all of the structural units (A) may be different, but all of the structural units (A) are preferably the same.

In the copolymer according to one of embodiments, the polycarbonate structural unit (B) means a structural unit formed from a polycarbonate structure, and may be a structural unit containing a plurality of carbonate units.

The polycarbonate structural unit is a polymer structural unit having a carbonate linkage in the main chain, and may be either an aliphatic polycarbonate structural unit or an aromatic polycarbonate structural unit, but is preferably an aliphatic polycarbonate structural unit.

Examples of aliphatic polycarbonate structural units include copolymer structural units such as polyethylene carbonate structural units, polypropylene carbonate structural units and polytrimethylene carbonate structural units, as well as derivative structural units in which side chains have been introduced into these types of structural units. Examples of aromatic polycarbonate structural units include polyaryl carbonate structural units and the like, and derivatives of these structural units. Examples of polyaryl carbonate structural units include polyphenyl carbonate structural units and the like.

In those cases where the copolymer contains two or more polycarbonate structural units (B), the polycarbonate structural units (B) contained in a single molecule of the copolymer may all be the same, or some or all of the structural units (B) may be different, but all of the structural units (B) are preferably the same.

The carbonate units that constitute the polycarbonate structural unit (B) may have a substituent. The polycarbonate structural unit (B) preferably has at least one carbonate unit having an alkoxyalkyloxycarbonyl group as a substituent. The carbonate units having this substituent preferably constitute one or more of the carbonate units in the polycarbonate structural unit (B), or may represent 80 mol% or more of all of the carbonate units that constitute the polycarbonate structural unit (B), or all of the carbonate units may have this substituent.

The alkoxyalkyloxycarbonyl group is preferably introduced by direct bonding to a carbon atom of a carbonate linkage in the main chain.

Further, in the alkoxyalkyloxycarbonyl group, the alkoxy group may be linear or branched, is preferably an alkoxy group of not more than 5 carbon atoms, more preferably an alkoxy group of not more than 3 carbon atoms, and even more preferably an ethoxy group or methoxy group, with a methoxy group being particularly preferred.

Furthermore, in the alkoxyalkyloxycarbonyl group, the alkylene group interposed between the alkoxy group and the carbonyl group may be linear or branched, preferably has not more than 5 carbon atoms and more preferably not more than 3 carbon atoms, and is even more preferably a propylene group or ethylene group, with an ethylene group being particularly preferred.

Specific examples of the alkoxyalkyloxycarbonyl group include a methoxyethyloxycarbonyl group, methoxypropyloxycarbonyl group, ethoxyethyloxycarbonyl group and ethoxypropyloxycarbonyl group, and a methoxyethyloxycarbonyl group is particularly preferred.

As a result of the copolymer having an alkoxyalkyloxycarbonyl group such as a methoxyethyloxycarbonyl group as a substituent of the polycarbonate structural unit (B), the adhesion to cells can be further enhanced, and cell differentiation during cell proliferation can be better suppressed. It is thought that by incorporating this substituent, the copolymer can more easily adopt a layer structure containing a water molecule between the molecular chains of water-insoluble structural units, with cells held stably in the polycarbonate structural unit (B) portion, meaning cell proliferation is better promoted and cell differentiation is better suppressed, although the present disclosure is not constrained by this theory.

In one preferred example, the polycarbonate structural unit (B) preferably contains at least one carbonate unit represented by general formula (II) shown below.

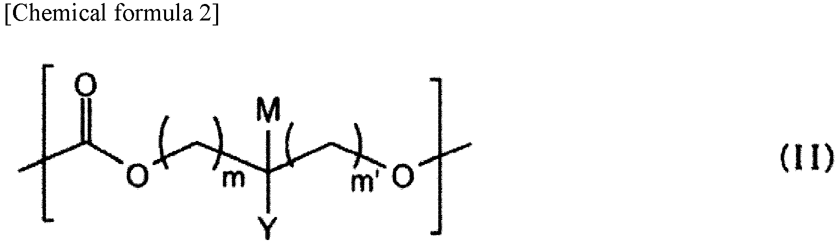

In general formula (II), M represents a hydrogen atom, a linear or branched alkyl group of not more than 5 carbon atoms, or a group represented by -L-Z, m and m' each independently represent an integer of 0 to 5, provided that m+m' = 1 to 7, Y is a group represented by -L-Z, L represents an alkylene group, ether linkage, ester linkage, single bond, -C(=O)-, or a divalent group having a combination of these moieties, and Z represents a chain-like ether group, cyclic ether group, group having an acetal structure, alkoxy group, alkoxyalkyl group, or a monovalent group having a combination of these groups.

The polycarbonate structural unit (B) may be a unit in which a plurality of carbonate units represented by general formula (II) are polymerized. In this case, the degree of polymerization of the units represented by general formula (II) may be, for example, within a range from 2 to 2,000.

The polycarbonate structural unit may contain at least one, or may contain two or more, of the carbonate units represented by general formula (II), and 80 mol% or more of all of the carbonate units included in the polycarbonate structural unit are preferably units represented by general formula (II). Moreover, all of the carbonate units included in the polycarbonate structural unit may be units represented by general formula (II).

Further, in those cases where the polycarbonate structural unit includes a plurality of units represented by general formula (II), the plurality of units represented by general formula (II) may all be the same, or some or all of the units may be different.

In general formula (1), M may represent a hydrogen atom, a linear or branched alkyl group of not more than 5 carbon atoms, preferably not more than 3 carbon atoms, and more preferably not more than 2 carbon atoms, or a group represented by -L-Z. Specific examples include a hydrogen atom, methyl group, ethyl group, propyl group, isopropyl group, butyl group, isobutyl group, tert-butyl group, sec-butyl group, pentyl group, isopentyl group, sec-pentyl group, and tert-pentyl group. Further, the group represented by -L-Z is as described below in relation to Y. Of the various possibilities, M is preferably a methyl group or ethyl group, and is more preferably a methyl group.

Further, m and m' each independently represent an integer of 0 to 5, and preferably satisfy m+m' = 1 to 7. Moreover, it is preferable that m and m' satisfy m+m' = 1 to 4, and more preferable that m=m'=1. In those cases where m=m'=1, the main chain of the polycarbonate structural unit has a polytrimethylene carbonate skeleton.

Furthermore, Y is a group represented by -L-Z, wherein L and Z are as described below.

L is a linker between the main chain and Z, and may be an alkylene group, ether linkage, ester linkage, single bond, -C(=O)-, or a divalent group having a combination of these moieties, and among these possibilities, is preferably an ether linkage, ester linkage, single bond, -C(=O)-, or a divalent group having a combination of these moieties, and is more preferably an ester linkage or a divalent group having -C(=O)-.

Z may be a chain-like ether group, cyclic ether group, group having an acetal structure, alkoxy group, alkoxyalkyl group, or a monovalent group having a combination of these groups, and among these possibilities, is preferably a chain-like ether group or an alkoxyalkyl group.

The chain-like ether group preferably has, for example, a structure composed of an alkylene glycol structure such as ethylene glycol or propylene glycol, or a polymer thereof.

Specific examples of Z groups of general formula (III) shown below in which R is an ethylene group or a propylene group, R' is a hydrogen atom or a linear or branched alkyl group of not more than 5 carbon atoms, and n represents an integer of 1 to 30.

-(R-O)n-R' (III)

In general formula (III), R is preferably an ethylene group. Further R' is preferably a methyl group or an ethyl group, and is more preferably a methyl group. Furthermore, n is preferably an integer of 1 to 5, and more preferably 1 or 2.

It is more preferable that Z is an alkoxyalkyl group, for example, a methoxyethyl group, methoxypropyl group, ethoxyethyl group, ethoxypropyl group, propyloxyethyl group or propyloxypropyl group, and of these, a methoxyethyl group is preferred.

Specific examples of the group represented by -L-Z include -OCH3, -OCH₂CH₃, - OCH₂CH₂CH₃, -CH₂OCH₃, -CH₂OCH₂CH₃, -CH₂OCH₂CH₂CH₃, -CH₂CH₂OCH₃, - CH₂CH₂OCH₂CH₃, -CH₂CH₂OCH₂CH₂CH₃, -CH₂OCH₂CH₂OCH₃, - CH₂OCH₂CH₂OCH₂CH₃, -CH₂OCH₂CH₂OCH₂CH₂CH₃, -CH₂CH₂OCH₂CH₂OCH₃, - CH₂CH₂OCH₂CH₂OCH₂CH₃, -CH₂CH₂OCH₂CH₂OCH₂CH₂CH₃, -C(=O)OCH₃, - C(=O)OCH₂CH₃, -C(=O)OCH₂CH₂CH₃, -C(=O)OCH₂CH₂OCH₃, - C(=O)OCH₂CH₂CH₂OCH₃, -C(=O)OCH₂CH₂OCH₂CH₃, - C(=O)OCH₂CH₂CH₂OCH₂CH₃, -C(=O)OCH₂CH₂OCH₂CH₂CH₃, and - C(=O)OCH₂CH₂CH₂OCH₂CH₂CH₃. Among these groups, -C(=O)OCH₃, - C(=O)OCH₂CH₃, -C(=O)OCH₂CH₂CH₃, -C(=O)OCH₂CH₂OCH₃, - C(=O)OCH₂CH₂CH₂OCH₃, -C(=O)OCH₂CH₂OCH₂CH₃, - C(=O)OCH₂CH₂CH₂OCH₂CH₃, -C(=O)OCH₂CH₂OCH₂CH₂CH₃ or - C(=O)OCH₂CH₂CH₂OCH₂CH₂CH₃ is preferred. Further, -C(=O)OCH₂CH₂OCH₃, - C(=O)OCH₂CH₂CH₂OCH₃, -C(=O)OCH₂CH₂OCH₂CH₃, - C(=O)OCH₂CH₂CH₂OCH₂CH₃, -C(=O)OCH₂CH₂OCH₂CH₂CH₃ or - C(=O)OCH₂CH₂CH₂OCH₂CH₂CH₃ is more preferred. The group -C(=O)OCH₂CH₂OCH₃ is particularly preferred.

In one specific preferred example, the polycarbonate structural unit (B) preferably contains at least one carbonate unit represented by general formula (I) shown below.

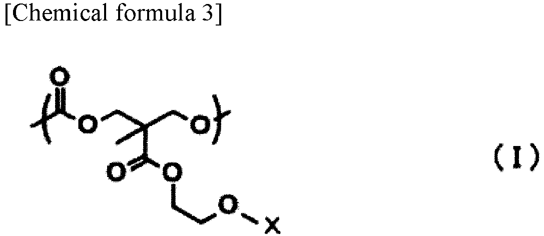

In general formula (I), X represents a hydrogen atom or an alkyl group of not more than 5 carbon atoms.

The alkyl group of not more than 5 carbon atoms for X may be a linear or branched group. Further, X is preferably a methyl group or ethyl group, and is more preferably a methyl group.

The polycarbonate structural unit (B) may be a unit in which a plurality of carbonate units represented by general formula (I) are polymerized. In this case, the degree of polymerization of the units represented by general formula (I) may be, for example, within a range from 2 to 2,000.

The polycarbonate structural unit may contain at least one, or may contain two or more, of the carbonate units represented by general formula (I), and 80 mol% or more of all of the carbonate units included in the polycarbonate structural unit are preferably units represented by general formula (I). Moreover, all of the carbonate units included in the polycarbonate structural unit may be units represented by general formula (I).

Further, in those cases where the polycarbonate structural unit includes a plurality of units represented by general formula (I), the plurality of units represented by general formula (I) may all be the same, or some or all of the units may be different.

The copolymer according to one of embodiments may be a block copolymer containing the polylactic acid structural unit (A) and the polycarbonate structural unit (B). In this case, the block structure of the block copolymer preferably has at least one terminal formed from the polylactic acid structural unit (A). In other words, the block structure of the block copolymer preferably has one terminal or both terminals formed from the polylactic acid structural unit (A). Examples of the block structure include AB-type, ABA-type and ABAB-type structures, and an ABA-type structure is preferred.

In the block structure of the block copolymer, by ensuring that one terminal or both terminals are formed from the polylactic acid structural unit (A), because the polylactic acid structural unit (A) exhibits favorable adhesion to the substrate, the cell scaffold material can be adhered more stably to the substrate.

Moreover, it is thought by incorporating the polylactic acid structural unit (A) at both terminals, such as in an ABA-type structure, the block copolymer can adhere to the substrate with the polylactic acid structural units (A) adhered to the substrate at both terminals of the block copolymer, and the intermediate polycarbonate structural unit (B) floating above the substrate. With this configuration, cells can be captured by the floating polycarbonate structural unit (B) portion, while the adhesion of the cells to the substrate is enhanced.

One example of the block copolymer is a copolymer represented by general formula (IV) shown below. More specifically, a copolymer represented by general formula (IV) shown below is preferred.

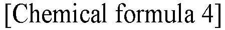

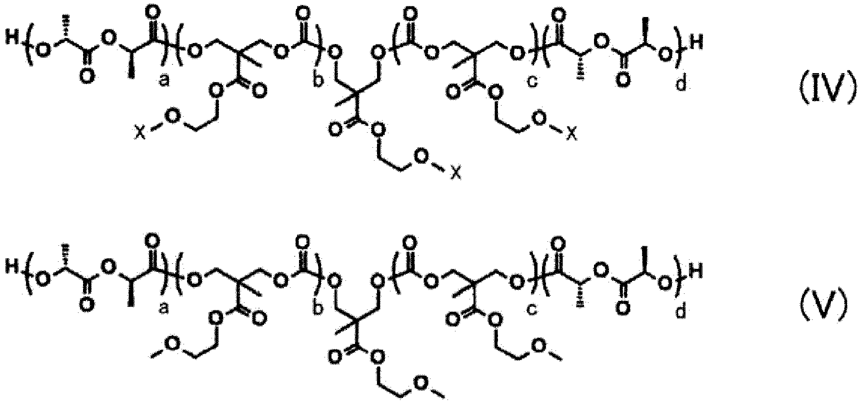

In general formula (IV), X is the same as described above for general formula (I), and therefore description here is omitted. In general formula (IV), a, b, c and d are numerical values that may be determined appropriately in accordance with the molecular weight of the copolymer, and these values may all be the same, or some or all of the values may be different. For example, it is preferable that one or both of a=d and b=c are satisfied, and more preferably that a=d and b=c.

In general formula (V), a, b, c and d are the same as described above for general formula (IV), and therefore descriptions here are omitted.

The number average molecular weight (Mn) of one molecule of the copolymer according to one of embodiments is preferably within a range from 5,000 to 100,000, more preferably from 10,000 to 80,000, even more preferably from 12,500 to 70,000, and particularly preferably from 15,000 to 50,000.

The molecular weight distribution (Mw/Mn) for one molecule of the copolymer according to one of embodiments is not particularly limited, but is, for example, typically not more than 2.0, and is preferably not more than 1.5, and more preferably 1.2 or less.

The number average molecular weight (Mn) of the polylactic acid structural unit (A) is preferably within a range from 1,000 to 50,000, more preferably from 3,000 to 40,000, even more preferably from 4,000 to 35,000, and still more preferably from 5,000 to 30,000.

Further, the molecular weight distribution (Mw/Mn) for the polylactic acid structural unit (A) is not particularly limited, but is preferably within a range from 1.0 to 10, more preferably from 1.0 to 8, and even more preferably from 1.05 to 5.

The number average molecular weight (Mn) of the polycarbonate structural unit (B) is preferably within a range from 2,000 to 50,000, more preferably from 5,000 to 40,000, even more preferably from 6,000 to 35,000, and still more preferably from 7,000 to 20,000.

Further, the molecular weight distribution (Mw/Mn) for the polycarbonate structural unit (B) is not particularly limited, but is preferably within a range from 1.0 to 10, more preferably from 1.0 to 8, and even more preferably from 1.05 to 5.

Here, the number average molecular weight of the polylactic acid structural unit (A) refers to the number average molecular weight of one block, and in those cases where the block copolymer includes two or more blocks of the polylactic acid structural unit (A), each block preferably satisfies the above range. This also applies to the polycarbonate structural unit (B).

Further, the number average molecular weight (Mn) and the weight average molecular weight (Mw) can be measured using gel permeation chromatography (GPC) calibrated against standard polystyrenes. Furthermore, the molecular weight distribution can be determined from the ratio (Mw/Mn) of the weight average molecular weight (Mw) relative to the number average molecular weight (Mn).

In the copolymer according to one of embodiments, it is preferable that all of the units that constitute the polylactic acid structural unit (A) are lactic acid units. Further, in the copolymer according to one of embodiments, it is preferable that all of the units that constitute the polycarbonate structural unit (B) are carbonate units.

In the copolymer according to one of embodiments, the total number of monomer units constituting the polylactic acid structural unit (A), relative to the total number of monomer units constituting the entire copolymer, is preferably at least 10 mol%, more preferably at least 30 mol%, and even more preferably 40 mol% or greater.

Further, in the copolymer according to another embodiment, the total number of monomer units constituting the polylactic acid structural unit (A), relative to the total number of monomer units constituting the entire copolymer, is preferably not more than 90 mol%, more preferably not more than 70 mol%, and even more preferably 60 mol% or less.

The total number of monomer units constituting the polylactic acid structural unit (A), relative to the total number of monomer units constituting the entire copolymer, is preferably within a range from 10 to 90 mol%, more preferably from 30 to 70 mol%, and even more preferably from 40 to 60 mol%.

In the copolymer according to one of embodiments, the total number of monomer units constituting the polycarbonate structural unit (B), relative to the total number of monomer units constituting the entire copolymer, is preferably at least 10 mol%, more preferably at least 30 mol%, and even more preferably 40 mol% or greater.

Further, in the copolymer according to another embodiment, the total number of monomer units constituting the polycarbonate structural unit (B), relative to the total number of monomer units constituting the entire copolymer, is preferably not more than 90 mol%, more preferably not more than 70 mol%, and even more preferably 60 mol% or less.

The total number of monomer units constituting the polycarbonate structural unit (B), relative to the total number of monomer units constituting the entire copolymer, is preferably within a range from 10 to 90 mol%, more preferably from 30 to 70 mol%, and even more preferably from 40 to 60 mol%.

In the copolymer according to one of embodiments, the molar ratio between the total number of monomer units constituting the polylactic acid structural unit (A) and the total number of monomer units constituting the polycarbonate structural unit (B) is preferably within a range from 10:90 to 90:10, more preferably from 30:70 to 70:30, and even more preferably from 40:50 to 50:40.

In the copolymer according to one of embodiments, the combined total of the total number of monomer units constituting the polylactic acid structural unit (A) and the total number of monomer units constituting the polycarbonate structural unit (B), relative to the total number of monomer units constituting the entire copolymer, is preferably at least 80 mol%, and more preferably 90 mol% or greater. Moreover, the copolymer according to one of embodiments may be composed only of the polylactic acid structural unit (A) and the polycarbonate structural unit (B).

In those cases where the copolymer according to one of embodiments is a block copolymer, wherein the block copolymer contains two or more blocks of the polylactic acid structural unit (A), it is preferable that the total number of monomer units constituting the two or more blocks of the polylactic acid structural unit (A) satisfies the range described above. This also applies to the polycarbonate structural unit (B).

One example of a method for synthesizing the copolymer according to one of embodiments is described below. The copolymer according to one of embodiments as described above is not limited to copolymers obtained using the following synthesis method.

The copolymer can be synthesized by preparing a polylactic acid and a polycarbonate, and then bonding the polylactic acid and the polycarbonate together.

Further, the copolymer can also be synthesized by preparing a polycarbonate, and then polymerizing and bonding a lactic acid, lactic acid lactide or combination thereof as a monomer at one terminal or both terminals of the polycarbonate.

Furthermore, the copolymer can also be synthesized by preparing a polylactic acid, and then polymerizing and bonding a carbonate monomer at one terminal or both terminals of the polylactic acid.

Moreover, the copolymer can also be synthesized by using a lactic acid and a carbonate, and performing either a random polymerization, or a block copolymerization using a polymerization reagent.

For the polylactic acid, poly-D-lactic acid, poly-L-lactic acid or poly-DL-lactic acid or the like may be used individually, or a combination of these compounds may be used, but the use of either poly-D-lactic acid or poly-L-lactic acid as a single component is preferred, and the use of poly-D-lactic acid as a single component is more preferred.

The number average molecular weight of the polylactic acid is preferably set accordingly in accordance with the target molecular weight of the structural unit (A) to be introduced into the block copolymer.

Examples of the monomer component that constitutes the polylactic acid include lactic acids, lactic acid lactides, and combinations of lactic acids and lactic acid lactides.

D-lactic acid, L-lactic acid or DL-lactic acid or the like may be used individually as the lactic acid monomer component that constitutes the polylactic acid, or a combination of these lactic acid compounds may be used, but the use of either D-lactic acid or L-lactic acid as a single component is preferred, and the use of D-lactic acid as a single component is more preferred.

D-lactic acid lactide, L-lactic acid lactide or DL-lactic acid lactide or the like may be used individually as the lactic acid lactide monomer component, or a combination of these lactic acid lactide compounds may be used, but the use of either D-lactic acid lactide or L-lactic acid lactide as a single component is preferred, and the use of D-lactic acid lactide as a single component is more preferred.

A combination of a lactic acid and a lactic acid lactide may also be used as the monomer component, but each compound may also be used individually.

An aliphatic polycarbonate or an aromatic polycarbonate may be used alone as the polycarbonate, or a combination of these types may be used.

A polycarbonate derivative containing at least one carbonate unit having an alkoxyalkyloxycarbonyl group as a substituent can be used favorably as the polycarbonate, and it is even more preferable that this substituent is a methoxyethyloxycarbonyl group.

Further, the use of a polycarbonate derivative containing at least one carbonate unit represented by general formula (II) as the polycarbonate is preferred. Moreover, the use of a polycarbonate derivative containing at least one carbonate unit represented by general formula (I) is also preferred. The carbonate unit represented by general formula (II) is preferably a carbonate unit having an alkoxyalkyloxycarbonyl group as a substituent. Further, the carbonate unit represented by general formula (I) is preferably a carbonate unit having a methoxyethyloxycarbonyl group as a substituent.

The number average molecular weight of the polycarbonate is preferably set accordingly in accordance with the target molecular weight of the polycarbonate structural unit (B) to be introduced into the block copolymer.

The carbonate monomer component that constitutes the polycarbonate is preferably an aliphatic carbonate, and examples include ethylene carbonate, propylene carbonate, and trimethylene carbonate and the like, as well as derivatives and the like of these compounds. These compounds may be used individually, or combinations two or more compounds may be used. A carbonate having a structure represented by general formula (II) or a cyclic carbonate that represents a cyclic derivative of such a compound is particularly preferred. Further, trimethylene carbonate derivatives described below may also be used.

Copolymerization of the polylactic acid or polylactic acid monomer component and the polycarbonate or polycarbonate monomer component is preferably conducted by solution polymerization, and if necessary, a polymerization initiator and/or polymerization catalyst or the like may be added to the synthesis system.

Examples of the polymerization solvent include dichloromethane, chloroform, diethyl ether, tetrahydrofuran (THF) and toluene.

Examples of the polymerization initiator include alcohol-based polymerization initiators such as 1-pyrenebutanol, lauryl alcohol, decanol and stearyl alcohol, and cyclic amine polymerization initiators such as 1,8-diazabicyclo[5.4.0]undec-7-ene (DBU), dimethylaminopyridine (DMAP), triethylenediamine (DABCO), (+)-sparteine, and (-)-sparteine.

Examples of the polymerization catalyst include difunctional thiourea compounds such as 1-(3,5-bis(trifluoromethyl)phenyl)-3-cyclohexyl-2-thiourea (TU).

The polymerization is preferably conducted at a temperature range from room temperature to any temperature that does not affect the synthesis system, and more specifically at a temperature within a range from room temperature to 50°C, either in an open atmosphere or under an inert atmosphere, and preferably under an inert atmosphere such as a nitrogen atmosphere, and is preferably conducted for a period from 1 minute to 12 hours, and more preferably a period from 30 minutes to 6 hours.

Completion of the polymerization reaction can be confirmed by whether or not the monomer components and the like are still present in the reaction system, and can be confirmed by methods such as ¹H-NMR and TLC. Once the polymerization reaction has progresses sufficiently, the polymerization reaction may be stopped by adding a reaction terminator. Examples of the reaction terminator include acetic acid, hydrochloric acid, sulfuric acid and benzoic acid.

A method for synthesizing a polycarbonate derivative having an alkoxyalkyloxycarbonyl group as a substituent is described below. This polycarbonate derivative can be obtained by ring-opening polymerization of a monomer having a functional group introduced as a side chain into a cyclic carbonate skeleton.

As a more specific example, a method for synthesizing a polycarbonate derivative containing a carbonate unit represented by general formula (II) is described below.

For example, in order to polymerize a polytrimethylene carbonate derivative having a unit represented by general formula (II) in which m=m'=1, a trimethylene carbonate derivative having a group represented by M and a group represented by Y introduced on a carbon atom of the trimethylene carbonate may be used as a monomer. M and Y are as described above in relation to general formula (II).

Specific examples of the trimethylene carbonate derivative include 5-methyl-5-(2-methoxyethyloxycarbonyl)-1,3-dioxan-2-one, 5-methyl-5-(2-ethoxyethyloxycarbonyl)-1,3-dioxan-2-one, 5-methyl-5-[2-(2-methoxyethoxy)ethyloxycarbonyl]-1,3-dioxan-2-one, 4-methyl-4-(2-methoxyethyloxycarbonyl)-1,3 -dioxan-2-one, 4-methyl-4-(2-ethoxyethyloxycarbonyl)-1,3-dioxan-2-one, and 4-methyl-4-[2-(2-methoxyethoxy)ethyloxycarbonyl]-1,3-dioxan-2-one.

In the synthesis of a polycarbonate derivative having a unit represented by general formula (II), the ring-opening polymerization of the monomer having a functional group introduced as a side chain into the cyclic carbonate skeleton can be conducted by any of various methods without any particular limitations. For example, the ring-opening polymerization may be conducted by a cationic polymerization reaction or an anionic polymerization reaction using any of various polymerization initiators, and if necessary, a polymerization catalyst or the like may also be used. There are no particular limitations on the polymerization solvent, the polymerization initiator and the polymerization catalyst, and the types of compounds described above in relation to synthesis of the above copolymer may be used.

More specifically, synthesis may be conducted in accordance with the method disclosed in JP 2014-161675 A.

The cell scaffold material according to one of embodiments may contain the copolymer according to the embodiment described above as the only component.

Further, the cell scaffold material according to one of embodiments, may also be provided in the form of a cell scaffold material composition containing an added solvent. Examples of the solvent include water, organic solvents, and mixed solvents thereof. The water may include not only water used as a solvent, but also intermediate water incorporated in the copolymer according to one of embodiments. Examples of the organic solvents include dichloromethane and methanol. The cell scaffold material composition may also contain, as necessary, medium additives described below and other additive components and the like.

Further, one of embodiments is able to provide a cell culture support containing the cell scaffold material according to one of embodiments described above and a substrate.

The cell culture support according to one of embodiments has the effects of suppressing cell differentiation and promoting cell proliferation, and can therefore be used favorably for growing cells of a specific differentiation stage by culturing.

There are no particular limitations on the shape of the substrate included in the support, and one or more shapes selected from the group consisting of flat plates, curved plates, spheres and blocks may be used.

Specifically, any of the materials typically used as cell culture substrates may be used as the substrate, and examples of substrates that may be used include dishes, well plates such as flat-bottom well plates and round-bottom well plates; cell containers such as Petri dishes; microbeads, microcarriers and three-dimensional blocks; and cell sheets and the like.

Although there are no particular limitations on the material of the substrate, a material that displays no cell toxicity is preferred. Examples of the material include resins including ester-based resins such as polyethylene terephthalate, (meth)acrylic-based resins, epoxy-based resins, urethane-based resins, styrene-based resins, thiol-based resins and silicone-based resins; metals such as pure nickel, titanium, platinum, gold, tungsten, rhenium, palladium, rhodium and ruthenium; alloys such as stainless steel, titanium/nickel, nitinol, cobalt/chromium and platinum/iridium alloys; and glass and ceramics and the like.

Furthermore, the substrate that has undergone cell culturing may be used in regenerative medicine where the substrate is transplanted into a living body. Examples of substrates that are suitable for regenerative medicine include biocompatible materials such as silicone, polyether block amides (PEBAX), polyurethane, silicone-polyurethane copolymers, ceramics, collagen, hydroxyapatite, nylon, polyethylene terephthalate, ultrahigh molecular weight polyethylenes such as Gore-Tex (a registered trademark), polyvinyl chloride, and other tissue-derived biomaterials; polylactide (PLA), polyglycolide (PGA), polycaprolactone (PCL) and copolymers of these polymers, PHB-PHV-based poly(alkanoic acids), polyesters, natural polymers such as starch, cellulose and chitosan, and derivatives of the above materials.

The cell culture support can be obtained by applying the cell scaffold material to the substrate.

For example, the cell scaffold material may be dissolved in an organic solvent, and the resulting liquid composition then applied to the substrate.

Examples of solvents that may be used include dichloromethane and methanol and the like. Further, in addition to the cell scaffold material, the liquid composition may also contain, medium additives described below and other additive components and the like. The amount of the cell scaffold material relative to the total mass of the liquid composition is preferably within a range from 0.05 to 10% by mass, and more preferably from 0.1 to 1% by mass.

There are no particular limitations on the method used for applying the liquid composition to the substrate, and for example, a method using spin coating may be used.

The amount of the cell scaffold material applied to the substrate may be adjusted appropriately in accordance with factors such as the type of substrate, the type of cell scaffold material, the type of cell used as the culture target, and the application method. The amount of the cell scaffold material applied to the substrate, expressed as a solid fraction amount per unit of surface area, may be set, for example, to a value within a range from 0.05 µg/mm² to 500 µg/mm². In those cases where an application method employing a spin coater is used, the solid fraction amount per unit of surface area, may be set, for example, to a value within a range from 0.01 µg/mm² to 10 µg/mm², to a value within a range from 0.05 µg/mm² to 5 µg/mm², or a value within a range from 0.1 µg/mm² to 3.0 µg/mm².

Before applying the cell scaffold material to the substrate, the substrate may be treated with another polymer besides the copolymer according to one of embodiments as described above. Examples of the other polymer include (meth)acrylic-based resins and the like. The other polymer is preferably combined with a suitable solvent, and applied to the substrate in liquid form. By interposing another polymer between the substrate and the cell scaffold material, adhesion of the scaffold material to the substrate can be enhanced.

The cell culture support according to one of embodiments may be placed in a culture system of the cells that are targeted for proliferation. Specifically, the cell culture support may be first placed in the medium, and the medium then inoculated with the target cells, or the cell culture support may be supplied to a medium in which the target cells already exist. As a result, the target cells and the cell culture support make contact, and the target cells adhere to the cell culture support and grow. Accordingly, by using the cell culture support according to one of embodiments, differentiation of the target cells can be suppressed and cell proliferation can be promoted.

A cell culture method according to one of embodiments is described below.

The cell culture method according to one of embodiments may include preparing a cell scaffold material, placing the cell scaffold material in a culture system, and culturing the cells in the presence of the cell scaffold material.

The cell scaffold material according to one of embodiments described above can be used as the cell scaffold material. As a result, cell differentiation can be suppressed, cell proliferation can be promoted, and a large number of undifferentiated cells can be manufactured.

In the step of preparing the cell scaffold material, the cell scaffold material according to one of embodiments described above is prepared. At this time, the cell scaffold material may be provided in the form of a composition containing the cell scaffold material as one component, or may be provided in the form of a cell culture support containing the cell scaffold material and a substrate.

In the step of placing the cell scaffold material in the culture system, a placement method is selected appropriately in accordance with the form in which the cell scaffold material is provided, and the cell scaffold material is placed in the culture system. For example, the cell scaffold material may be placed in the culture system prior to cell inoculation in the form of a cell scaffold material composition or a cell culture support containing a plate-shaped substrate. In an alternative method, the culture system may first be inoculated with the target cells, and the cell scaffold material then subsequently placed in the culture system as a cell scaffold material composition or a cell culture support in the form of microbeads.

In the step of conducting culturing of the cells, culturing of the target cells is conducted in the presence of the cell scaffold material.

Examples of cells that may be used as the culture target include primary culture cells, cultured cell lines, and recombinant culture cell lines and the like. There are no particular limitations on the cell origins, and examples include mammals such as humans, chimpanzees, monkeys, cows, horses, pigs, dogs, cats, rabbits, rats, mice and hamsters; and birds such as chickens. Further, cells produced by hybridization of two or more different types of cells may also be used.

There are no particular limitations on the organ or tissue from which the cells are derived, and examples include the blood and lymphatic system, vascular system, cranial nervous system, bone marrow, muscle tissue, thymus gland, salivary gland, oral cavity, esophagus, stomach, liver, gallbladder, spleen, small intestine, large intestine, rectum, skin, cornea, lungs, thyroid, mammary gland, uterus, cervix, ovaries, testes, pancreas, kidneys, adrenal cortex, bladder, placenta, umbilical cord, embryo, fetus, tail, mesenchymal stem cells, and cancer cells and the like.

Furthermore, stem cells can be used favorably as the cells to be cultured. Examples include stem cells having pluripotency such as embryonic stem cells (ES cells), induced pluripotent stem cells (iPS cells), embryonic carcinoma cells (EC cells), embryonic germ cells (EG cells), nuclear transfer ES cells and somatic cell-derived ES cells; tissue stem cells such as hematopoietic stem cells, bone marrow-derived mesenchymal stem cells, adipose tissue-derived mesenchymal stem cells, umbilical cord-derived mesenchymal stem cells, stem cells derived from other stromal tissue, Muse cells and neural stem cells; and various types of stem cells such as precursor cells and fibroblasts found in various tissues such as the liver, pancreas, adipose tissue, bone tissue, cartilage tissue and nerve tissue.

The cell culturing may employ the types of conditions typically used for the culturing of target cells, with the specific conditions selected in accordance with the type of cells being cultured.

There are no particular limitations on the medium used for the cell culture, provided the cells can survive and proliferate, and the medium may be selected appropriately in accordance with the type of cells being cultured.

Either serum media or serum-free media may be used as the medium, but the cell scaffold material according to one of embodiments can be used favorably for culturing in a serum-free medium.

Examples of serum-free media include Eagle Medium, Dulbecco's Modified Eagle Medium (low-glucose or high-glucose), Eagle MEM Medium, αMEM Medium, IMDM Medium, Ham's F10 Medium, Ham's F12 Medium, RPMI-1640 Medium, and blended media of these media.

Serum media can be prepared by adding a serum to a serum-free medium. In those cases where a serum is added to a serum-free medium, serums such as Fetal bovine serum (FBS), horse serum and human serum may be used. When a serum is added, the serum concentration is preferably not higher than 30%.

Additives may be added to the medium if necessary. Examples of these additives include vitamins such as vitamin A, vitamin B1, vitamin B2, vitamin B6, vitamin B12, vitamin C and vitamin D; coenzymes such as folic acid; amino acids such as glycine, alanine, arginine, asparagine, glutamine, isoleucine and leucine; sugars and organic acids that acts as carbon sources such as lactic acid; growth factors such as EGF, FGF, PFGF and TGF-β; interleukins such as IL-1 and IL-6; cytokines such as TNF-α, TNF-β and leptin; metal transporters such as transferrin; metal ions such as iron ions, selenium ions and zinc ions; SH reagents such as β-mercaptoethanol and glutathione; and proteins such as albumin.

There are no particular limitations on the cell culture method, and a method suited to the particular target cell may be used. The cell culture may be conducted within a temperature range from 30 to 40°C, and preferably at a temperature of 37°C, at a humidity within a range from 70 to 100%, and preferably within a range from 95 to 100%, and in an atmosphere containing 2% to 7% CO₂, and preferably 5% CO₂. There are no particular limitations on the cell subculture timing or method, and a method suited to the target cell may be used.

The culture configuration may be either a two-dimensional culture in which cell adhesion is easier, or a three-dimensional culture in which the cells are suspended in a culture system. The culture configuration may be selected appropriately in accordance with the type of cell and the medium configuration and the like.

Further, according to one of embodiments, a cell culture kit containing the cell scaffold material according to one of embodiments described above, a substrate and a medium may be provided. In this kit, the cell scaffold material according to one of embodiments, the substrate and the medium may each be stored in a separate container. Furthermore, by using a cell scaffold support of one of embodiments containing a substrate and a cell scaffold material, the cell culture support and the medium may each be stored in a separate container. Furthermore, these kits may also include the cells to be cultured. Further, these kits may also include implements or the like to be used in the culturing process. The cell scaffold material and cell scaffold material support according to one of embodiments are as described above.

Further, one of embodiments can provide use of a copolymer having a polylactic acid structural unit (A) and a polycarbonate structural unit (B) for a cell scaffold material.

Furthermore, one of embodiments can provide use of a copolymer having a polylactic acid structural unit (A) and a polycarbonate structural unit (B) for the production of a cell scaffold material.

Here, details relating to the copolymer having a polylactic acid structural unit (A) and a polycarbonate structural unit (B) are as disclosed in the items described above relating to the copolymer in the cell scaffold material according to one of embodiments described above. For example, in the copolymer, the polycarbonate structural unit (B) may include at least one unit having an alkoxyalkyloxycarbonyl group as a substituent, or the polycarbonate structural unit (B) may include at least one unit represented by general formula (I) shown above. Furthermore, in the copolymer, the polylactic acid structural unit (A) may be a poly-D-lactic acid structural unit or a poly-L-lactic acid structural unit. Moreover, the copolymer may be an ABA-type block copolymer. An arbitrary combination of these items is also possible. In one example, in the copolymer, the polycarbonate structural unit (B) either includes at least one unit having an alkoxyalkyloxycarbonyl group as a substituent, or includes at least one unit represented by general formula (I) shown above, and the polylactic acid structural unit (A) is a poly-D-lactic acid structural unit or a poly-L-lactic acid structural unit. The copolymer of this example may be an ABA-type block copolymer.

### EXAMPLES

The present disclosure is described below in further detail using a series of examples, but the present disclosure is not limited to these examples.

### [Methods for Evaluating Physical Properties]

The molecular weight, structural confirmation, and degree of polymerization progression for each of the products obtained in the examples were evaluated using the following procedures.

### (1) Molecular Weight

The number average molecular weight (Mn) and the weight average molecular weight (Mw) were measured by connecting GPC columns (Gelpack GL-R420, R430, R440, manufactured by Hitachi Chemical Techno Service, Ltd.) to a Chromaster GPC analysis system (manufactured by Hitachi High-Tech Science Corporation), and eluting the sample at 1.75 mL/min using THF as the eluent.

### (2) Molecular Weight Distribution (Mw/Mn)

The molecular weight distribution was determined as the ratio (Mw/Mn) between the values for the weight average molecular weight (Mw) and the number average molecular weight (Mn) determined using the method described above in (1).

### (3) NMR Measurement

Structural analyses of the monomers and polymers were conducted by ¹H-NMR measurements using an NMR measurement apparatus (JEOL 500 MHz JNM-ECX, manufactured by JEOL Ltd.). Chemical shifts were recorded relative to CDCl₃ (¹H: 7.26 ppm).

### [Reagents]

The reagents 2,2-bis(methylol)propionic acid (bis-MPA: 98%), 2-methoxyethanol (99.8%) and Amberlyst-15 (a registered trademark) (dry, moisture ≤ 1.5%) were procured from Sigma-Aldrich Japan KK, and used without modification. Ethyl acetate (99.5%), dichloromethane (DCM: 99.5%), pyridine (99.5%), ammonium chloride (98.5%), sodium bicarbonate (99.5 to 100.3%), 1,8-diazabicyclo[5.4.0]undec-7-ene (DBU: 99.0%), benzoic acid (99.5%), diethyl ether (99.0%), hexane (95.0%), trimethylene carbonate (TMC: 98.0%), methanol (99.8%), tetrahydrofuran (THF: 99.5%), (+)-sparteine (Sp), and 2-propanol (99.7%) were procured from Kanto Chemical Co., Inc. Anhydrous grade THF and DCM (water content < 10 ppm) were supplied from a solvent supply system from Kanto Chemical Co., Inc. Hydrochloric acid (35.0 to 37.0% by mass) and magnesium sulfate (95.0%) were procured from FUJIFILM Wako Pure Chemical Corporation, and triphosgene (98%), benzyl alcohol, 2-methoxyethyl acrylate (>98.0%), 1,4-dioxane (>99.0%) and 2,2-azobis(isobutyronitrile) (AIBN, >98.0%) were procured from Tokyo Chemical Industry Co., Ltd., and each compound was used without modification. Further, 1-(3,5-bis(trifluoromethyl)phenyl)-3-cyclohexyl-2-thiourea (TU) was synthesized with reference to the method previously disclosed. The monomers and TU were dissolved in THF and dried using CaH₂ (calcium hydride). The DBU was subjected to distillation under reduced pressure using CaH₂ prior to use.

The D-lactic acid lactide (D-lactide) was procured from Purac NV.

Lipidure (a registered trademark)-CM5206 (hereafter also abbreviated as PMB) was procured from NOF Corporation.

### [Synthesis Example 1: Synthesis of Polylactic Acid-Polycarbonate Block Copolymer (PDMED)]

### <Synthesis of 2-methoxyethyl 2,2-bis(hydroxymethyl)propanoate (ME-MPA)>

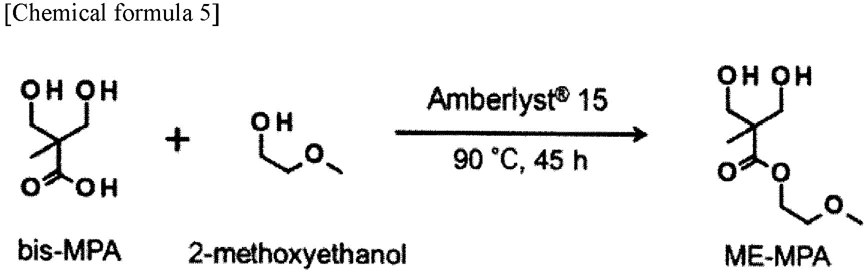

First, bis-MPA (30.0 g, 0.224 mol) and an ion exchange resin Amberlyst-15 (a registered trademark) (6.00 g) were added to 2-methoxyethanol (300 mL, 3.82 mol), and the resulting mixture was stirred under heating at 90°C for 48 hours. Subsequently, the ion exchange resin was removed from the reaction solution by filtration, and the obtained filtrate was concentrated under reduced pressure. The product was then dried under vacuum to obtain ME-MPA as a light yellow oily substance (41.4 g, yield: 96.3%).
¹H-NMR (400 MHz, CDCl₃): δ 4.35 (t, J=4.8 Hz, 2H), 3.85 (d, J=12 Hz, 2H), 3.73 (d, J=12 Hz, 2H), 3.63 (t, J=5.0 Hz, 2H), 3.39 (s, 3H), 1.11 (s, 3H)

### <Synthesis of 2-(2-methoxyethyloxycarbonyl)-2-methyltrimethylene carbonate (MEMTC)>

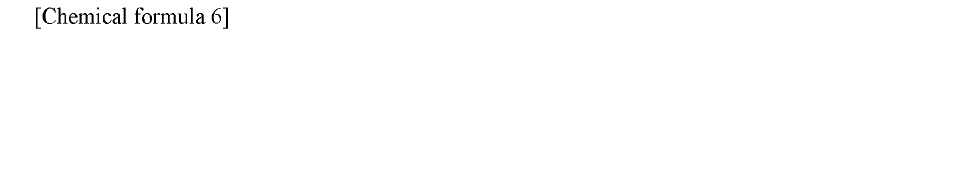

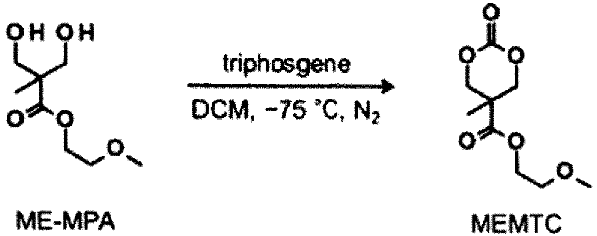

ME-MPA (20.0 g, 0.104 mol) and pyridine (50.5 mL, 0.624 mol) were added to dichloromethane (DCM) (120 mL), and the resulting mixture was cooled to -75°C in a dry ice-2-propanol (IPA) bath. Subsequently, a DCM solution (160 mL) of triphosgene (15.4 g, 0.0520 mol) was added dropwise to the reaction mixture, and the resulting mixture was then stirred under cooling at -75°C for one hour, and then at room temperature for a further two hours. Following completion of the reaction, a saturated aqueous solution of ammonium chloride (200 mL) was added and stirred for 30 minutes, and the organic phase was then washed twice with a 1 N aqueous solution of hydrochloric acid (200 mL), with a saturated aqueous solution of sodium bicarbonate (200 mL), with a saturated saline solution (200 mL), and finally with ion-exchanged water (200 mL). The thus obtained organic phase was dried over magnesium sulfate, and then concentrated and dried under reduced pressure. Subsequently, the product was purified by column chromatography (ethyl acetate), yielding MEMTC as a colorless viscous liquid (14.1 g, yield: 46.2%).

¹H-NMR (400 MHz, CDCl₃): δ 4.68 (d, J=11 Hz, 2H), 4.32 (t, J=9.5 Hz, 2H), 4.20 (d, J=11 Hz, 2H), 3.57 (t, j=4.8 Hz, 2H), 3.33 (s, 3H), 1.31 (s, 3H)

### <Synthesis of poly[2-(2-methoxyethyloxycarbonyl)-2-methyltrimethylene carbonate] (PMEMTC)>

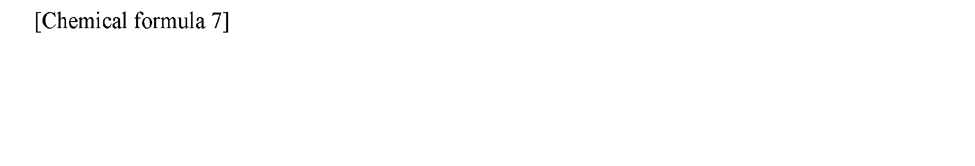

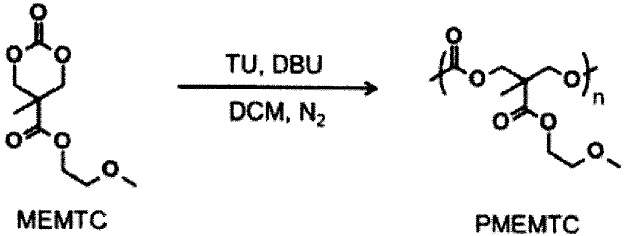

In a glovebox under a nitrogen atmosphere, MEMTC (0.433 g, 1.99 mmol), TU (15.0 mg, 0.041 mmol) and DBU (6.1 mg, 0.040 mmol) were stirred in DCM (2 mL) at room temperature . After two hours of reaction, consumption of the monomers was confirmed by ¹H-NMR, a few drops of benzoic acid were added as a terminator, and the mixture was stirred overnight. Subsequently, the reaction solution was re-precipitated in 2-propanol (60 mL), and the product was dried under vacuum to obtain a colorless, viscous polymer PMEMTC (0.325 g, yield: 75.1%).
GPC: Mn 8,700, Mw/Mn 1.11
¹H-NMR (400 MHz, CDCl₃): δ 4.30 (m, 6H), 3.58 (t, J=4.8 Hz, 2H), 3.36 (s, 3H), 1.27 (s, 3H)

### <Synthesis of PDLA-PMEMTC-PDLA (PDMED)>

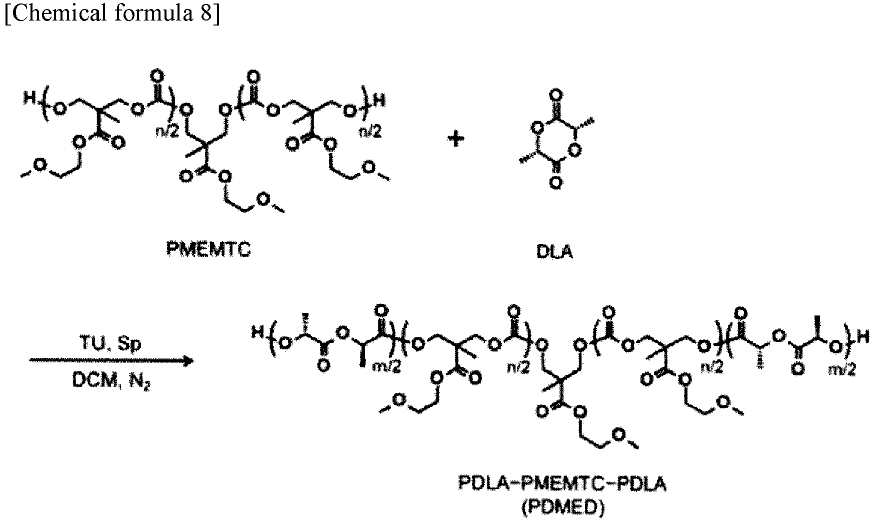

In a glovebox under a nitrogen atmosphere, PMEMTC (0.16 g, 0.734 mmol) was dissolved in DCM (480 mg), a small amount of calcium hydride was added as a desiccant, and the resulting mixture was stirred for one hour. Subsequently, the mixture was filtered through a syringe filter, and the filtrate was added dropwise to a vial containing (+)-sparteine (Sp) (3.44 mg, 1.49 mmol). In a separate preparation, TU (5.8 mg, 1.49 mmol) and D-lactide (DLA, 77.1 mg, 0.535 mmol) were dissolved in DCM (240 mg), and the resulting solution was then mixed with the initially prepared solution and stirred at room temperature. After two hours of reaction, consumption of the monomers was confirmed by ¹H-NMR, a few drops of benzoic acid were added as a terminator, and the mixture was stirred for several hours. Subsequently, the reaction solution was re-precipitated in 2-propanol (30 mL), and the product was dried under vacuum to obtain a white solid PDLA-PMEMTC-PDLA (PDMED) (0.132 g, yield: 55%).
GPC: Mn 18,000, Mw/Mn 1.10
¹H-NMR (400 MHz, CDCl₃): δ 5.26 to 5.12 (q, 2H), 4.30 (m, 6H), 3.58 (t, J=4.8 Hz, 2H), 3.36 (s, 3H), 1.70 to 1.50 (d, 6H), 1.27 (s, 3H)

### [Synthesis Example 2: Synthesis of Polylactic Acid (PDLA)]

TU (5.8 mg, 1.49 mmol) and D-lactide (77.1 mg, 0.535 mmol) were dissolved in DCM (240 mg), and the resulting solution was stirred at room temperature. After two hours of reaction, the reaction solution was re-precipitated in 2-propanol (30 mL), and the product was dried under vacuum to obtain a white solid PDLA.

### [Synthesis Example 3: Synthesis of PTMC (polytrimethylene carbonate)]

Benzyl alcohol (4.32 mg, 0.04 mmol), trimethylene carbonate (2.01 g, 20 mmol), DBU (182.4 mg, 1.4 mmol) and TU (447.1 mg, 1.4 mmol) were dissolved in DCM (20 mL) and the solution was stirred. After 24 hours, benzoic acid was added to halt the reaction, the reaction solution was re-precipitated in 2-propanol, and the supernatant liquid was removed by centrifugal separation. The thus obtained viscous product was recovered by dissolution in DCM, and then concentrated and dried using an evaporator, thus obtaining PTMC.

### [Synthesis Example 4: Synthesis of PMEA (poly(2-methoxyethyl)acrylate)]

First, 15 g of 2-methoxyethyl acrylate (130.14 g/mol, 115 mmol) was dissolved in 60 g of 1,4-dioxane, and the solution was subjected to nitrogen bubbling for 30 minutes. Next, 15 mg of AIBN (0.091 mmol) as an initiator was dissolved in a small amount of 1,4-dioxane and added to the reaction solution, and a polymerization was conducted at 75°C for 6 hours while nitrogen bubbling was continued. Subsequently, the polymer produced in the polymerization was collected as a precipitate by adding the polymerization solvent medium dropwise to 1,000 ml of n-hexane. The obtained by-product was purified by repeating the precipitation operation three times using a THF/n-hexane system. The purified polymer was recovered by dissolution in THF, concentrated using an evaporator, and then dried under vacuum at 60°C for 30 hours, thus obtaining PMEA.

### [Methods for Evaluating Cell Cultures]

The evaluations described below were conducted using the PDMED obtained in Synthesis Example 1, the PMEMTC that represents an intermediate in Synthesis Example 1, the PDLA obtained in Synthesis Example 2, the PTMC obtained in Synthesis Example 3, the PMEA obtained in Synthesis Example 4, and PMB (LIPIDURE (a registered trademark) CM5206, manufactured by NOF Corporation) as polymers.

### (1) Preparation of Cell Scaffold Material

A PET film (Diafoil T100E125 E07, manufactured by Mitsubishi Chemical Corporation) of thickness 125 µm that had been punched out into a circular shape with a diameter of 15 mm was subjected to a degreasing treatment by immersion overnight in methanol. Subsequently, each polymer was dissolved in either DCM or methanol and adjusted to a concentration of 0.2% by mass. Next, 100 µL samples of this polymer solution were applied twice to the treated PET film using a spin coater, and following standing for one day at room temperature, the coated film was washed by immersion overnight in pure water. Following drying, the polymer-coated PET film (hereafter referred to as a support) was sterilized, inside a clean bench, by irradiation for 10 minutes with a spot UV irradiation device (SP-11, manufactured by Ushio Inc.) at an output of 4 W/cm². Further, an untreated support (PET support) was prepared as a control, by conducting the degreasing treatment, but then not coating the PET film with a polymer.

### (2) Model Protein Adsorption Test

A PET film (Diafoil T100E125 E07, manufactured by Mitsubishi Chemical Corporation) of thickness 125 µm that had been punched out into a circular shape with a diameter of 15 mm was subjected to a degreasing treatment by immersion overnight in methanol. Next, 100 µL samples of a solution prepared by dissolving 0.1% by mass BSA (bovine serum albumin) in a phosphate buffer solution (PBS, pH 7.4) containing 1% by mass of Triton X-100 were applied twice to the back surface of the substrate using a spin coater, and following standing for one day at room temperature, the coated film was washed by immersion overnight in pure water and then dried, thus preventing non-specific adsorption to the PET. Subsequently, each polymer was dissolved in either DCM or methanol and adjusted to a concentration of 0.2% by mass. Next, 100 µL samples of this polymer solution were applied twice to the top surface of the treated PET film using a spin coater, and following standing for one day at room temperature, the coated film was washed by immersion overnight in pure water.

The thus obtained support was placed in a 24-well plate designed for cell culturing (Coster 24 wells, manufactured by Corning Inc.), a fluorescent-labeled BSA (FITC labeling) was dissolved in 0.1 w/v% PBS and added to each well in an amount of 500 µL/well, and the well plate was shaken (tilt angle: 6°, 30 r/min) at 37°C for 60 minutes. An untreated support (PET support) that had not been coated with the polymer was used as a control, and was treated in the same manner as above.

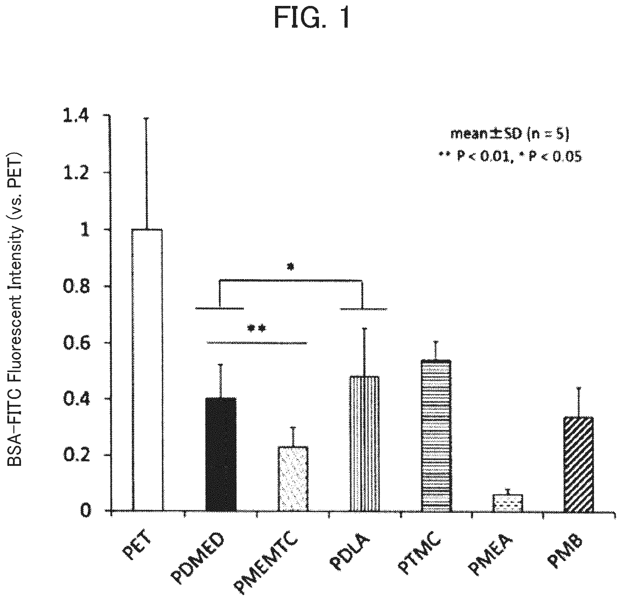

Following shaking, the cells were washed with PBS, the state of adsorption was observed using a fluorescence microscope (BZ-X, manufactured by Keyence Corporation), and the amount of adsorption was evaluated from the fluorescent intensity. The results are shown in FIG. 1. FIG. 1 is a graph illustrating the relative fluorescent intensity of the fluorescent-labeled BSA when supports coated with each of the various polymers were used, using the fluorescent intensity of the PET support as a standard.

### (3) Cell Culture Tests

### (3-1) Normal Human Fibroblasts

The PDMED support, PMB support and PET support prepared using the above (1) Preparation of Cell Scaffold Material were set on the bottom surface of each well of a 24-well tissue culture plate. Further, a well containing no set support was also prepared. Each well was inoculated with normal human dermal fibroblasts (NHDF) at a seeding density of 5×10³ cells/cm². A medium prepared by adding 10% fetal bovine serum (FBS) to Eagle MEM medium was added to each well.

This plate was cultured at 37°C under a 5% CO₂ atmosphere. One hour, one day, two days, three days and seven days after starting the cell culture, the state of the cells were inspected, and at each time, 1,000 µL of the culture supernatant was extracted and subjected to centrifugal separation at 7,000 rpm for one minute, with 500 µL of the resulting supernatant then being extracted and supplied to a quantitative evaluation for FGF-2 (fibroblast growth factor 2).

### (i) Method for Evaluating Cell Proliferation

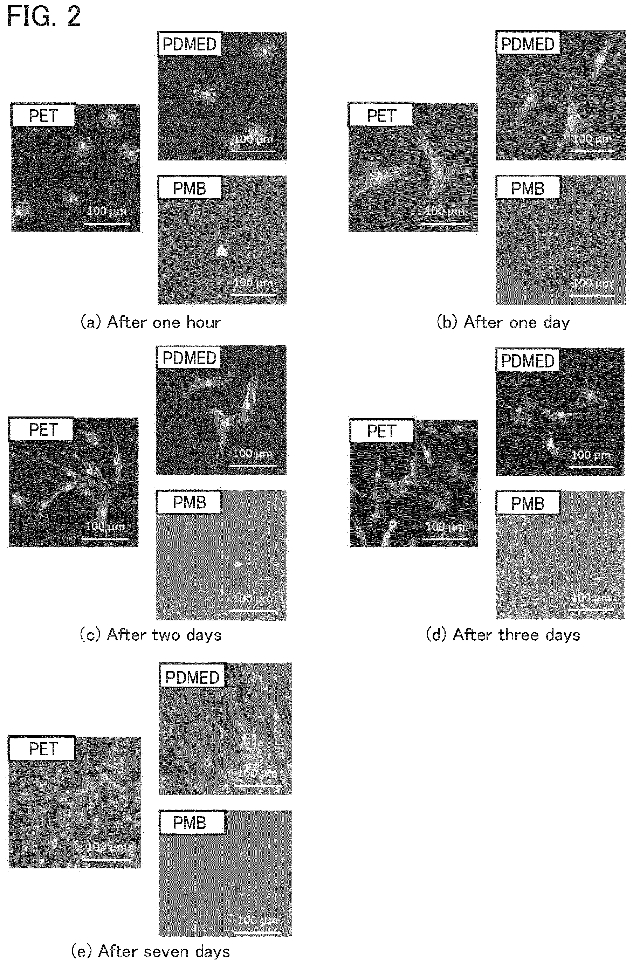

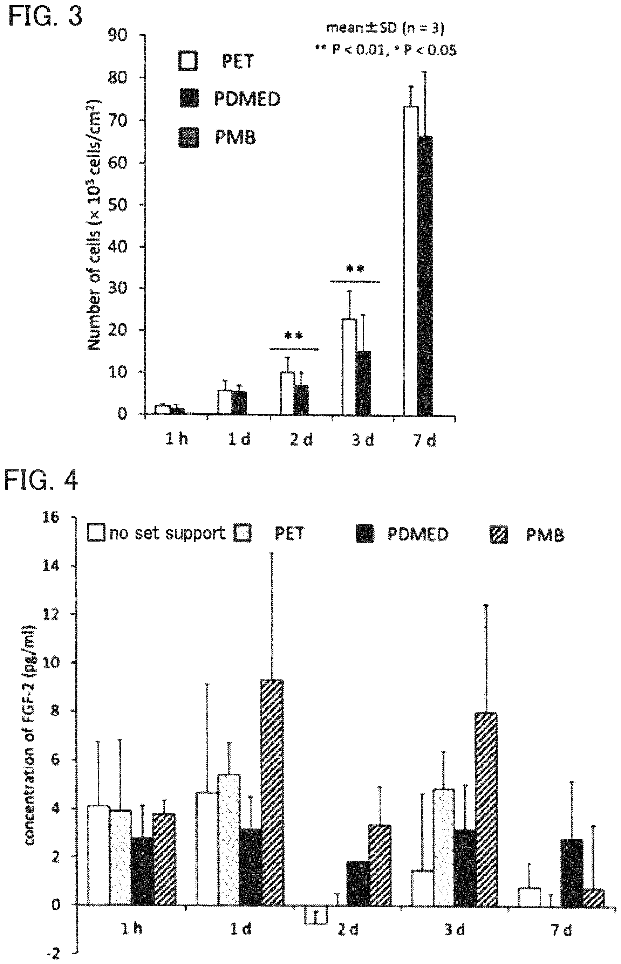

Using a fluorescence difference microscope (MVX10, manufactured by Olympus Corporation), the state of cell proliferation was observed, and the number of cells was measured. FIG. 2 is a series of photographs viewed using the fluorescence difference microscope, showing the states (a) one hour after the start of cell culturing, (b) one day after the start of cell culturing, (c) two days after the start of cell culturing, (d) three days after the start of cell culturing, and (e) seven days after the start of cell culturing. FIG. 3 is a graph showing the number of cells relative to the cell culture time.

### (ii) Method for Evaluating Cell Differentiation Suppression

The culture supernatants sampled at the various cell culture times were each measured using a human FGF-2 measurement ELISA kit (manufactured by R&D Systems, Inc.). FIG. 4 is a graph showing the FGF-2 concentration relative to the cell culture time.

Based on the cell observations conducted at each of the culture times, it was evident that although the numbers of cells in the plate using PDMED (hereafter referred to as PDMED wells) were lower than the plate using PET (hereafter referred to as the PET wells) that was used as a blank, in general, a favorable level of proliferation was maintained. In contrast, in the case of the plate using PMB (hereafter referred to as PMB wells), it was evident that cells did not adhere, and the polymer had almost no contribution to cell proliferation. When the state of the cells was observed, cell proliferation with considerable spreading was observed in the PET wells, and when the cell density increased, proliferation of cell masses was observed, which is a feature of fibroblast proliferation. In the PDMED wells, cell spreading was minimal, and even when the cell density increased, it was confirmed that the cells proliferated while remaining adhered to the support, without forming masses.

Based on the FGF-2 quantitative results, it was evident that in the PMB wells in which the cells were not adhered to the support, a large amount of FGF-2 was released in the initial stages of the culture. In the PET wells, there was considerable fluctuation in the release of the factor depending on the state of proliferation, and in particular, on the seventh day, release of the factor had stopped due to mass formation. On the other hand, it was evident that the PDMED wells exhibited a stable level of factor release throughout the culture.

Observations of the cell proliferation also suggested that in the base of PDMED, only cell proliferation was occurring preferentially, with the proliferation of cell masses that represents a function of human fibroblasts not observed. This is also clear from the stable release of FGF-2.

### (3-2) iPS Cells

Using the same method as that described above in (1) Preparation of Cell Scaffold Material, a PDMED support, PMB support, an untreated PET support as a control, and a laminin (iMatrix 511, manufactured by Nippi Inc.) support as a control were each prepared with a support diameter of 50 mm. Each support was set on the bottom surface of a 60 mm dish (3010-060, manufactured by Iwaki Co., Ltd.). Each dish (referred to as the PDMED dish, PMB dish, PET dish, and iMatrix dish respectively) was inoculated with feeder-free iPS cells from Kyoto University in an amount of 2.5×10⁴ cells/dish.

A medium (StemFit AK02, manufactured by Ajinomoto Healthy Supply Co., Inc.) was added to each dish, and culturing was conducted at 37°C under a 5% CO₂ atmosphere. After seven days of cell culture, an inverted microscope (CKX31SF, manufactured by Olympus Corporation) was used to observe the state of the cells, and undifferentiated cells were separated and quantified by FACS (flow cytometry). Further, in a similar manner to (3-1) above, from the start of culturing through to the seventh day, samples of the culture supernatant were extracted and supplied to IL-6 (interleukin-6) and TNFα (tumor necrosis factor α) quantity evaluations. Each of the evaluations was conducted using an ELISA kit (manufactured by R&D Systems, Inc.).

The undifferentiated cell rate on the seventh day of culture and the concentrations of IL-6 and TNFα on the fifth day of culture are shown in Table 1.

An undifferentiated cell rate (%) closer to 100 (%) means better suppression of cell differentiation. Furthermore, an IL-6 concentration closer to 0 means better suppression of cell differentiation. Moreover, a TNFα concentration closer to 0 means better suppression of cell differentiation.

**[Table 1]**

| Item | PET | PMB | PDMED | iMatrix |
|---|---|---|---|---|
| Undifferentiated cell rate (%) | 97.1 | 98.8 | 99.8 | 90.2 |
| IL-6 (pg/mL) | 0.06 | 0.33 | below detection limit | 9.58 |
| TNFα (pg/mL) | below detection limit | below detection limit | below detection limit | 1.20 |

IL-6 is known as a cytokine associated with differentiation induction. These test results revealed that in the case of the iMatrix dish which acted as one control, the undifferentiated cell rate of the viable cells was lower than the other dishes, and the levels of IL-6 and TNFα release on the fifth day of culture were much higher than the other dishes. Based on these results, it was evident that cell differentiation was not adequately suppressed in the iMatrix dish.

Further, in the PMB dish and the PET dish, the undifferentiated cell rate on the seventh day of culture was lower than that of the PDMED dish, and on the fifth day of culture, IL-6 release was also confirmed. In the case of the PMB dish, TNFα release of about 2 pg/mL was also confirmed on the second day of culture (data not shown). These results indicated that even in the PMB dish and the PET dish, cell differentiation was not adequately suppressed.

In contrast to these cases, in the PDMED dish, the undifferentiated cell rate on the seventh day of culture was high, and no release of either cytokine was detected from the start of culturing through to the fifth day. Based on these results, it was evident that when PDMED was used as the cell scaffold material, a contribution to proliferation could be achieved while suppressing cell differentiation.

The disclosure of the present disclosure is related to the subject matter disclosed in prior Japanese Application 2019-120033 filed on June 27, 2019, the entire content of which is incorporated by reference herein. It should be noted that, besides those already mentioned above, many modifications and variations may be made to the above embodiments without departing from the novel and advantageous features of the present disclosure. Accordingly, all such modifications and variations are intended to be included within the scope of the appended claims.

## Claims

1. A cell scaffold material comprising a copolymer having a polylactic acid structural unit (A) and a polycarbonate structural unit (B).

2. The cell scaffold material according to Claim 1, wherein the polycarbonate structural unit (B) includes at least one unit having an alkoxyalkyloxycarbonyl group as a substituent.

3. The cell scaffold material according to Claim 1 or 2, wherein the polycarbonate structural unit (B) includes at least one unit having a methoxyethyloxycarbonyl group as a substituent.

4. The cell scaffold material according to Claim 1, wherein the polycarbonate structural unit (B) includes at least one unit represented by general formula (I) shown below: 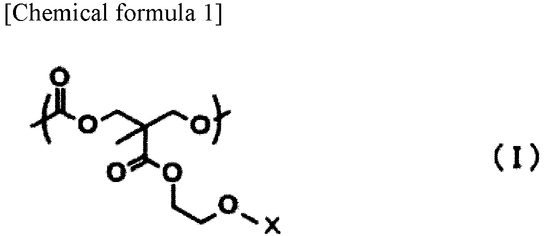 wherein in general formula (I), X represents a hydrogen atom or an alkyl group having not more than 5 carbon atoms.

5. The cell scaffold material according to Claim 4, wherein X in the general formula (I) is a methyl group.

6. The cell scaffold material according to any one of Claims 1 to 5, wherein the copolymer is a block copolymer in which one terminal or both terminals have the polylactic acid structural unit (A).

7. The cell scaffold material according to Claim 6, wherein the copolymer is an AB-type, ABA-type or ABAB-type block copolymer.

8. The cell scaffold material according to Claim 7, wherein the copolymer is an ABA-type block copolymer.

9. The cell scaffold material according to any one of Claims 1 to 8, wherein the polylactic acid structural unit (A) is a poly-D-lactic acid structural unit or a poly-L-lactic acid structural unit.

10. A cell culture support comprising the cell scaffold material according to any one of Claims 1 to 9, and a substrate.

11. A cell culture method that comprises: preparing the cell scaffold material according to any one of Claims 1 to 9, placing the cell scaffold material in a culture system, and culturing cells in the presence of the cell scaffold material.

12. The cell culture method according to Claim 11, wherein the culture system comprises a serum-free medium.
